# EUROPEAN PATENT APPLICATION

(11) **EP 4 783 194 A1**
(43) Date of publication of application: **29.07.2026**
(21) Application number: 26153211.3
(22) Date of filing: 21.01.2026
(51) Int. Cl.: G16H 50/30, A61B 5/00, G16H 40/63

(54) **DYNAMIC DETERMINATION OF PATIENT TILE DISPLAY FORMATS**

(30) Priority: 23.01.2025 US 202563748833 P
(71) Applicant: Welch Allyn, Inc., Skaneateles Falls, NY 13153 (US)
(72) Inventor: Myers, Thomas Arthur, Skaneateles Falls, 13153 (US)
(74) Representative: Reddie & Grose LLP

(57) **Abstract**

A central monitoring system associated with a healthcare environment displays, in a user interface, patient tiles associated with corresponding patients. The patient tiles display information provided by monitoring devices that are respectively associated with the patients within the healthcare environment. The central monitoring system dynamically determines display formats, indicating dimensions, detail levels, and/or other visual attributes, for the patient tiles individually. The central monitoring system dynamically determines a display format for an individual patient tile based on an acuity level of the corresponding patient, based on types of monitoring devices used to monitor the patient, based on types of data provided by the monitoring devices, and/or based on other factors.

## Description

The present disclosure relates to patient monitoring, and more particularly to dynamically selecting a display format for presentation of patient monitoring data based on one or more monitoring devices.

Monitoring devices may be used to monitor one or more patients in a hospital or other healthcare environment. For example, blood pressure monitors, heart rate monitors, and/or other types of monitoring devices may be set up to measure or determine patient vital signs and/or other information about patients.

Medical staff may use patient information provided by the monitoring devices to diagnose the patients, to determine how to treat the patients, to monitor conditions of the patients over time, and/or for other purposes. However, in an environment in which multiple patients are being monitored by monitoring devices, it may be difficult to present data from multiple monitoring devices and/or about multiple patients in a format that allows a viewer to quickly and easily understand and interpret that data.

As an example, some systems are configured to display information about each of a set of multiple patients with a consistent layout, and/or with a consistent level of detail, in a user interface. Accordingly, it may be difficult for a viewer of the user interface to, at a glance, quickly discern from such uniformly-presented information which of the patients have the most severe conditions.

As another example, if multiple monitoring devices provide data about the same patient, some systems may be configured to present all of that data within a user interface. Accordingly, the user interface may become crowded and overly detailed, such that it may be difficult for a viewer of the user interface to, at a glance, identify or discern relevant data about the patient. Other systems may be configured to display information from only one monitoring device at a time in a user interface, such that a viewer may be unable to see relevant information about a patient that was provided by another monitoring device.

The example systems and methods described herein may be directed toward mitigating or overcoming one or more of the deficiencies described above.

A central monitoring system associated with a healthcare environment may receive patient monitoring information, associated with a set of patients, from monitoring devices within the healthcare environment that have been configured to monitor the patients. The central monitoring system may display, in a user interface, patient tiles that are associated with corresponding patients and that present information provided by the monitoring devices. The central monitoring system may dynamically determine display formats, indicating dimensions, detail levels, and/or other visual attributes, for the patient tiles individually. The central monitoring system may dynamically determine display formats for patient tiles based on acuity levels of the corresponding patients. Accordingly, patient tiles for patients with more severe conditions and higher acuity levels may be displayed within the dashboard with larger dimensions and/or higher detail levels than other patient tiles for patients with less severe conditions and lower acuity levels. A viewer may infer at a glance, based on the dimensions and/or detail levels of the patient tiles, the acuity levels of the patients associated with the patient tiles. The central monitoring system may also, or alternately, dynamically determine display formats for patient tiles based on other factors, such as types of monitoring devices used to monitor the patients, based on amounts of monitoring devices used to monitor the patients, based on types of data provided by the monitoring devices, and/or based on other factors.

The techniques described herein may relate to a central monitoring system associated with a healthcare environment. The central monitoring system includes a dashboard, presented in a user interface displayed via a computing system including a processor, configured to display a plurality of patient tiles. The plurality of patient tiles is associated with a plurality of patients present within the healthcare environment. An individual patient tile of the plurality of patient tiles corresponds to an individual patient of the plurality of patients, and displays data elements indicated by patient monitoring data provided by at least one monitoring device associated with the individual patient within the healthcare environment. The central monitoring system also includes a display format determiner, executed by the computing system, configured to dynamically determine display formats used to display the plurality of patient tiles in the dashboard. The display format determiner determines a display format for the individual patient tile based at least in part on an acuity level of the individual patient.

The techniques described herein may relate to a dashboard, presented via a user interface of a central monitoring system associated with a healthcare environment. The dashboard includes a first patient tile associated with a first patient. The first patient tile is configured to present first data elements indicated by first patient monitoring data received by the central monitoring system from at least one first monitoring device associated with the first patient within the healthcare environment. The first patient tile is displayed within the dashboard using a first display format dynamically determined by a computing system associated with the central monitoring system based at least in part on a first acuity level of the first patient. The dashboard also includes a second patient tile, associated with a second patient. The second patient tile is configured to present second data elements indicated by second patient monitoring data received by the central monitoring system from at least one second monitoring device associated with the second patient within the healthcare environment. The second patient tile is displayed within the dashboard using a second display format dynamically determined by the computing system based at least in part on a second acuity level of the second patient.

The techniques described herein may relate to a computer-executed method. The computer-executed method includes receiving, by a central monitoring system executed by a computing system including a processor, patient monitoring data associated with a patient in a healthcare environment from at least one monitoring device. The computer-executed method also includes determining, by the central monitoring system, an acuity level of the patient. The acuity level indicates a current severity of at least one of a medical condition or illness of the patient. The computer-executed method also includes determining, by the central monitoring system, and based at least in part on the acuity level of the patient, a display format for a patient tile associated with the patient that presents at least one data element indicated by the patient monitoring data. The computer-executed method also includes causing, by the central monitoring system, the patient tile to be displayed in a user interface dashboard of the central monitoring system based on the display format.

The invention will now be further described with reference to the accompanying figures, in which: in the figures, the left-most digit(s) of a reference number identifies the figure in which the reference number first appears. The use of the same reference numbers in different figures indicates similar or identical items or features.
FIG. 1 shows an example of a central monitoring system in use within a healthcare environment.
FIG. 2 shows examples of different display formats that may be used to present patient tiles within the dashboard of the central monitoring system.
FIG. 3 shows a flowchart illustrating an example method for dynamically determining a display format for a patient tile in the dashboard of the central monitoring system.
FIG. 4 shows a flowchart illustrating an example method for dynamically determining a display format for a patient tile in the dashboard, based at least in part on types of patient monitoring data associated with a corresponding patient.
FIG. 5 shows a flowchart illustrating an example method for dynamically determining a display format for a patient tile in the dashboard, based at least in part on changes to a set of monitoring devices associated with a corresponding patient.
FIG. 6 shows an example system architecture for a computing system that may execute one or more elements described herein, such as one or more elements of the central monitoring system.

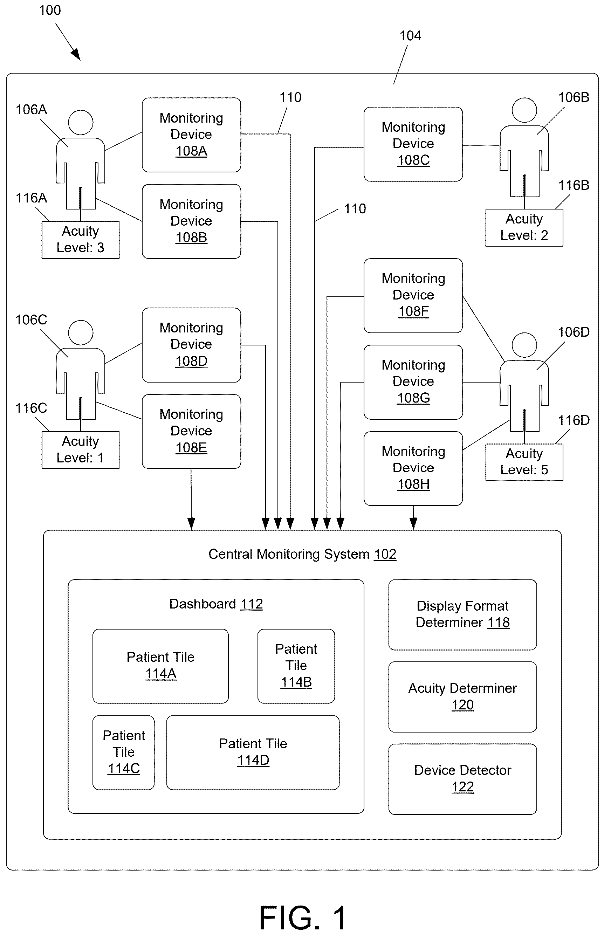

FIG. 1 shows an example 100 of a central monitoring system 102 in use within a healthcare environment 104. The healthcare environment 104 may be in, or be associated with, a hospital, medical clinic, or other facility or environment where patients 106 are treated and/or monitored. As an example, a set of patients 106, including a first patient 106A, a second patient 106B, a third patient 106C, and a fourth patient 106D, may be treated in a hospital wing or other healthcare environment 104, as shown in FIG. 1.

As discussed further below, monitoring devices 108 present in the healthcare environment 104 may be configured to measure and/or otherwise determine patient monitoring data 110 about the patients 106, such as one or more types of vital signs, metrics, or other types of data. The monitoring devices 108 may provide the patient monitoring data 110 to the central monitoring system 102, for instance via wired and/or wireless data connections. The central monitoring system 102 may have a user interface that is configured to present information about the patients 106, such as patient monitoring data 110 received from the monitoring devices 108, other information associated with or derived from the patient monitoring data 110, and/or other information about the patients 106.

The user interface of the central monitoring system 102 may display, for instance via a dashboard 112, patient tiles 114 that are associated with respective patients 106. The dashboard 112 may be a page, view, or other user interface display or presentation that presents one or more patient tiles 114. Patient tiles 114 may be sections, areas, boxes, windows, or other elements within the dashboard 112 or other user interface of the central monitoring system 102. The dashboard 112 or other user interface of the central monitoring system 102 may concurrently present multiple distinct patient tiles 114 that are respectively associated with corresponding patients 106. The patient tiles 114 may individually display information about respective patients 106, such as patient monitoring data 110 received from monitoring devices 108 and/or other data about the patients 106.

For instance, FIG. 1 shows a non-limiting example in which the dashboard 112 concurrently displays a first patient tile 114A that presents information about the first patient 106A, a second patient tile 114B that presents information about the second patient 106B, a third patient tile 114C that presents information about the third patient 106C, and a fourth patient tile 114D that presents information about the fourth patient 106D. Although FIG. 1 shows an example with four patients 106 and four patient tiles 114, in other examples any other larger or smaller number of patients 106 may be present in the healthcare environment 104, and the dashboard 112 may accordingly display a larger or smaller number of distinct patient tiles 114 that correspond with those patients 106.

As described further below, the central monitoring system 102 may dynamically determine display formats for the patient tiles 114. The central monitoring system 102 may determine the display format for each patient tile 114 individually, such that different patient tiles 114 may be displayed using the same display format or different display formats. In some examples, the central monitoring system 102 may determine the display formats for respective patient tiles 114 based on acuity levels 116 that indicate severities of corresponding patients' illnesses or medical conditions. In other examples, the central monitoring system 102 may also, or alternately, determine the display formats for respective patient tiles 114 based on other factors associated with the corresponding patients 106, such as amounts of monitoring devices 108 associated with individual patients 106, types of monitoring devices 108 associated with the individual patients 106, types of patient monitoring data 110 associated with the individual patients 106 and/or provided by respective monitoring devices 108, and/or other factors.

The monitoring devices 108 may be configured to measure and/or otherwise determine patient monitoring data 110 associated with respective patients 106. The monitoring devices 108 may include electrocardiogram (ECG) monitors, blood pressure monitors, pulse rate (PR) monitors, blood oxygen saturation (SPO2) monitors, carbon dioxide (CO2) monitors, blood glucose monitors, thermometers, scales or other weight monitors, activity monitors, fall detection monitors, and/or other types of monitoring devices 108.

In some examples, a monitoring device 108 may be a dedicated device that is configured to determine a particular type of patient monitoring data 110 about a patient 106. As a non-limiting example, a monitoring device 108 may be a blood pressure monitor designed specifically to measure a patient's blood pressure.

However, other monitoring devices 108 may have multiple types of sensors and/or may be configured to determine multiple types of patient monitoring data 110 about a patient 106. As a non-limiting example, a monitoring device 108 may be a hospital bed that includes various sensors and/or systems that are configured to determine multiple types of patient monitoring data 110 about a patient 106 that is in the hospital bed, such as the patient's weight, the patient's heart rate, the patient's respiration patterns, the patient's movement patterns, and/or other types of data about the patient 106.

A monitoring device 108 may be configured to measure or determine one or more corresponding types of patient monitoring data 110 about a patient 106 on a continuous basis, on a periodic, episodic, or occasional basis, on demand, in response to trigger conditions, or based on any other schedule, basis, or monitoring frequency. Different monitoring devices 108 may be configured to determine patient monitoring data 110 at the same times, or at different times or on different schedules. As an example, an ECG monitor may be configured to continuously detect a waveform and/or other information indicative of activity of a patient's heart, while a blood pressure monitor may be configured to measure a patient's blood pressure on demand or on a relatively infrequent episodic basis, such as once per hour or on any other non-continuous basis.

Within the healthcare environment 104, different patients 106 may be respectively monitored by different sets of monitoring devices 108. Each set of monitoring devices 108 may contain one or more monitoring devices 108. For instance, in the example shown in FIG. 1, monitoring device 108A and monitoring device 108B may be associated with the first patient 106A, monitoring device 108C may be associated with the second patient 106B, monitoring device 108D and monitoring device 108E may be associated with the third patient 106C, and monitoring device 108F, monitoring device 108G, and monitoring device 108H may be associated with the fourth patient 106D.

In some examples, different patients 106 may be monitored by the same number and/or types of monitoring devices 108. However, in other examples, different sets of monitoring devices 108 that are associated with different patients 106 may include different numbers of monitoring devices 108 and/or different types of monitoring devices 108.

As a non-limiting example, an ECG monitor and a thermometer may be set up to monitor the first patient 106A, while a blood pressure monitor may be set up to monitor the second patient 106B. In this example, the second patient 106B may not be monitored by an ECG monitor or a thermometer. However, in another non-limiting example a first ECG monitor and a first thermometer may be set up to monitor the first patient 106A, while a second ECG monitor and/or a second thermometer may be set up to monitor the second patient 106B instead of or in addition to a blood pressure monitor.

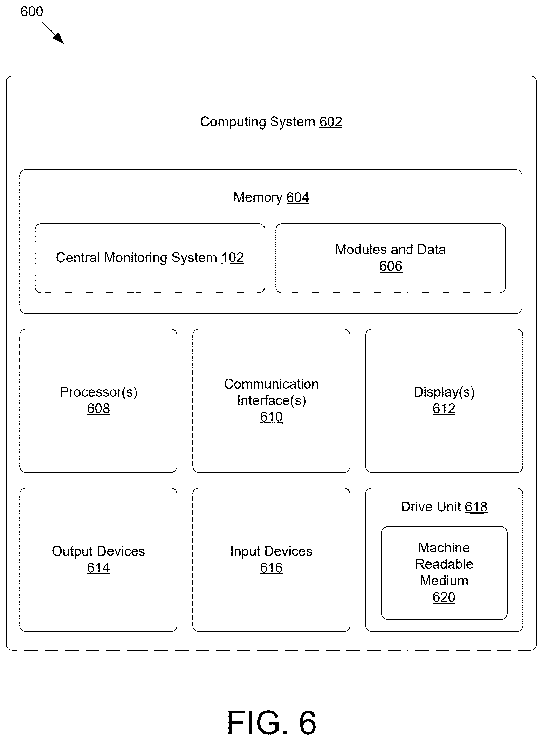

The central monitoring system 102 associated with the healthcare environment 104 may receive patient monitoring data 110 from the monitoring devices 108. The central monitoring system 102 may be executed by one or more computing systems associated with the healthcare environment 104. In some examples, one or more elements of the central monitoring system 102 may be executed by a local computing system that is present on site at the healthcare environment 104. In other examples, one or more elements of the central monitoring system 102 may be executed by a remote computing system, such as a cloud computing system or a server located in a different building or another environment that is remote from the healthcare environment 104. A computing system that may execute one or more elements of the central monitoring system 102 is shown in FIG. 6, and is described further below with respect to that figure.

The central monitoring system 102 may receive patient monitoring data 110, determined by the monitoring devices 108, from the monitoring devices 108 via wired and/or wireless data communications. For instance, some monitoring devices 108 may be configured to transmit patient monitoring data 110 to the central monitoring system 102 via Ethernet cables or other wired data connections. Other monitoring devices 108 may be configured to wirelessly transmit patient monitoring data 110 to the central monitoring system 102 via a Wi-Fi network, via cellular data connections, via Bluetooth connections, or via other types of wireless data connections.

The user interface of the central monitoring system 102 may display patient monitoring data 110 received from the monitoring devices 108, information derived from the patient monitoring data 110, and/or other information about respective patients 106. The user interface of the central monitoring system 102 may be presented via a screen or other visual display. As described herein, the dashboard 112 that presents distinct patient tiles 114 associated with respective patients 106 may be displayed via the user interface of the central monitoring system 102.

The dashboard 112 and other user interface elements of the central monitoring system 102 may be displayed via a dedicated screen associated with the central monitoring system 102, and/or via other devices or systems that log in to, or otherwise access, the central monitoring system 102. As a non-limiting example, the dashboard 112 of the central monitoring system 102 may be displayed via one or more screens at a nurses station or other central location within the healthcare environment 104. As another non-limiting example, the dashboard 112 of the central monitoring system 102 may be displayed via one or more user devices. For instance, in some examples the dashboard 112 of the central monitoring system 102 may be accessed and/or presented via a website, an executable client application, and/or other systems, such that one or more users may access and view the dashboard 112 of the central monitoring system 102 via corresponding user devices such as laptop computers, tablet computers, smartphones, and/or other devices.

Accordingly, if a set of multiple patients 106 is being treated in the healthcare environment 104, the dashboard 112 of the central monitoring system 102 may be displayed to users associated with the healthcare environment 104 via one or more screens at a nurses station and/or other locations within the healthcare environment 104, and/or via one or more user devices. Nurses, doctors, and/or other staff members or users associated with the healthcare environment 104 may view the dashboard 112 of the central monitoring system 102 to see information about any or all of the patients 106 being treated in the healthcare environment 104 via corresponding patient tiles 114.

A user may view the dashboard 112 at a single location to quickly discern information about multiple patients 106 at the same time via the patient tiles 114, without physically visiting different locations of different patients 106 at different times within the healthcare environment 104. Although staff may physically visit and treat patients 106 individually within the healthcare environment 104, the dashboard 112 may display a set of patient tiles 114 that collectively present information about multiple patients 106 concurrently via a centralized platform.

The dashboard 112 of the central monitoring system 102 may accordingly allow staff or other users to remotely monitor patients 106 that may not need immediate treatment, to remotely determine which patients 106 do need immediate treatment, to determine how to prioritize treatment of different patients 106, and/or for other purposes. For instance, such users may view information presented via the patient tiles 114, displayed via the dashboard 112 of the central monitoring system 102, to determine current statuses of the patients 106, to determine how medical conditions of the patients 106 are progressing, to determine which patients 106 should be visited next by staff members, and/or for other purposes.

Information displayed in the dashboard 112, via the patient tiles 114, may be based on patient monitoring data 110 received from multiple monitoring devices 108. The dashboard 112 may accordingly display aggregated information from multiple monitoring devices 108, including multiple types of monitoring devices 108 and/or monitoring devices 108 provided by different manufacturers or vendors. The dashboard 112 may also display information provided by monitoring devices 108 that were not originally designed to provide information to a centralized system that may receive data from more than one monitoring device 108 or multiple types of monitoring devices 108.

As a non-limiting example, a monitoring device 108 may be a pulse monitor that was originally designed with an expectation that pulse data for a single patient 106, measured by the pulse monitor, would be displayed in isolation on a dedicated pulse monitor display. However, in this example the pulse monitor may be configured to provide measured pulse data to the central monitoring system 102, instead of or in addition to a dedicated pulse monitor display. Accordingly, the pulse data measured by the pulse monitor may be displayed in a patient tile 114 via the dashboard 112 of the central monitoring system 102, potentially alongside one or more other types of data provided by one or more other monitoring devices 108 that are also associated with the same patient 106, and/or potentially on the same screen as data for other patients 106 presented via other patient tiles 114 in the dashboard 112.

As another non-limiting example, monitoring devices 108 produced by different manufacturers may be set up to monitor the same patient 106, and may be configured to provide corresponding patient monitoring data 110 to the central monitoring system 102. Accordingly, even if the monitoring devices 108 produced by the different manufacturers are not directly compatible and/or use different data types, the central monitoring system 102 may be configured to receive and interpret patient monitoring data 110 about the same patient 106 from the different monitoring devices 108, and to make the monitoring data 110 from the different monitoring devices 108 available for display within the same patient tile 114. Patient monitoring data 110 from monitoring devices 108 that are not otherwise compatible, and/or that are not configured to communicate directly with each other, may nevertheless be displayed in the same patient tile 114.

Overall, data elements indicated by patient monitoring data 110, provided by sets of monitoring devices 108 associated with corresponding patients 106, may be displayed concurrently via patient tiles 114 of the dashboard 112. However, as described herein, different patient tiles 114 may be displayed using different display formats, or the same display formats, concurrently within the dashboard 112.

The central monitoring system 102 may have a display format determiner 118 that dynamically determines display formats for the patient tiles 114 within the dashboard 112. The display formats may indicate sizes and/or dimensions of the patient tiles 114, detail levels of the patient tiles 114, types of data elements to be displayed within the patient tiles 114, layout arrangements or positions for one or more types of data elements to be displayed within the patient tiles 114, and/or other attributes of visual presentations of the patient tiles 114 within the dashboard 112. In some examples, the display formats may also indicate whether user-selectable UI options or controls associated with monitoring devices 108 are to be displayed within corresponding patient tiles 114.

The display format determiner 118 may determine display formats for each patient tile 114 individually, such that the display format determiner 118 may determine that two different patient tiles 114 should be displayed using the same display format or using different display formats. As a non-limiting example, the central monitoring system 102 may determine that the first patient tile 114A should have larger dimensions, and/or should present a higher level of detail, than the second patient tile 114B within the dashboard 112. However, the central monitoring system 102 may also determine that another pair of patient tiles 114 should be displayed using the same dimensions and with the same level of detail within the dashboard 112.

In some examples, dimensions and/or a level of detail indicated by a display format for a patient tile 114 may indicate how many, and/or which, data elements are to be displayed in the patient tile 114 based on patient monitoring data 110 received from one or more monitoring devices 108. Patient monitoring data 110 received from one or more monitoring devices 108 may indicate multiple data elements, such as charts, values, or other information. Such data elements may be displayed, or hidden from view, within a patient tile 114 on an individual basis. Accordingly, if a patient tile 114 is being displayed with relatively large dimensions and/or a high level of detail, there may be room in the patient tile 114 to display all or most of the data elements indicated by the patient monitoring data 110. However, if a patient tile 114 is being displayed with relatively small dimensions and/or a low level of detail, some or all of the data elements indicated by the patient monitoring data 110 may be hidden and not displayed within the patient tile 114. The patient tile 114 and/or other elements of the dashboard 112 or user interface may have user-selectable options that allow users to selectively access and/or view data elements indicated by received patient monitoring data 110 that are not displayed within the patient tile 114, for instance via a "view more details" option.

The display format determiner 118 may determine the display format for a patient tile 114 based on one or more factors, such as the acuity level 116 of the corresponding patient 106, the amount of monitoring devices 108 associated with the corresponding patient 106, the types of monitoring devices 108 associated with the corresponding patient 106, types of patient monitoring data 110 associated with the corresponding patient 106, and/or other factors. The display format determiner 118 may use such factors associated with different patient tiles 114 to dynamically determine respective display formats for the different patient tiles 114.

As such factors change over time with respect to a particular patient tile 114, the display format determiner 118 may adjust, dynamically and/or in real-time, the display format used to present that particular patient tile 114 in the dashboard 112. As a non-limiting example, if the acuity level 116B of the second patient 106B increases over time and/or additional monitoring devices 108 are added to monitor the second patient 106B, the display format determiner 118 may dynamically change the display format of the second patient tile 114B within the dashboard 112 so that the second patient tile 114B is larger, more detailed, and/or displays different amounts or types of data about the second patient 106B.

As discussed above, in some examples the display format determiner 118 may dynamically determine display formats of respective patient tiles 114 based on acuity levels 116 associated with corresponding patients 106. An acuity level 116 for a particular patient 106 may indicate a severity of that patient's illness or medical condition. Differences between acuity levels 116 of different patients 106 may thus indicate relative differences in the severity levels of those patients' illnesses or conditions. For instance, a patient 106 with a life-threatening condition may have a higher acuity level 116 than another patient 106 with a minor injury. Accordingly, the display format determiner 118 may determine that a patient tile 114 associated with the patient 106 with the life-threatening condition and the higher acuity level 116 should be displayed in the dashboard 112 with larger dimensions, and/or with more detail, than a different patient tile 114 for the patient 106 with the minor injury and the lower acuity level 116.

Acuity levels 116 of patients 106 may be indicated via numerical values, tiers, and/or via other types of indicators. As an example, acuity levels 116 of patients 106 may be indicated as numerical values on a scale of 1-5, 1-10, 1-100, or any other scale. In some examples, higher numerical values on such a scale may indicate more severe illnesses or medical conditions, and thus higher acuity levels 116, than lower numerical values. However, in other examples lower numerical values on such a scale may indicate more severe illnesses or medical conditions, and thus higher acuity levels 116, than higher numerical values. As another example, acuity levels 116 of patients 106 may be indicated using a tier system. For instance, a tier system may include three tiers, such as a "high acuity" tier, a "medium acuity" tier, and a "low acuity" tier, or may have any other number of tiers that indicate respective acuity levels 116.

In some examples, an acuity level 116 of a patient 106 may be different from an early warning score (EWS) that the healthcare environment 104 associates with that patient 106. An EWS score may indicate whether a patient's condition is likely to worsen in the future, but the EWS score may be distinct from the patient's acuity level 116 that indicates the current severity of the patient's condition. For instance, a high EWS score may indicate that the patient's acuity level 116 is likely to increase in the future, even if the patient's acuity level 116 is currently relatively low. In some examples a patient's EWS score may be a factor used to determine the patient's acuity level 116, for instance along with one or more other factors, but in other examples the patient's acuity level 116 may be determined via one or more other factors without use of the patient's EWS score.

In the healthcare environment 104, it may be useful for staff and/or other users of the central monitoring system 102 to be able to quickly determine current acuity levels 116 of individual patients 106, and/or to quickly determine how acuity levels 116 of different patients 106 compare to each other. For instance, because patients 106 with higher acuity levels 116 may have more severe medical conditions than other patients 106 with lower acuity levels 116, it may be useful for staff in the healthcare environment 104 to be able to quickly determine which patients 106 have higher acuity levels 116 than other patients 106 so that treatment of those higher-acuity patients 106 may be prioritized.

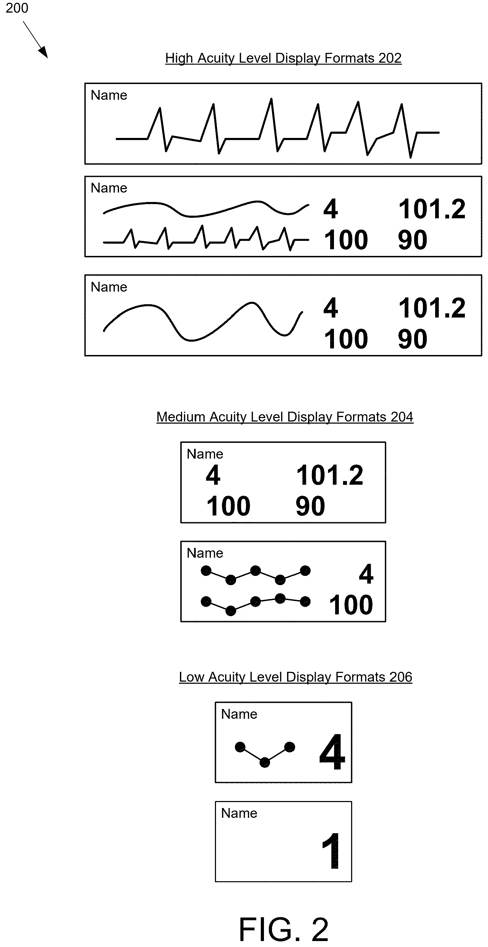

The central monitoring system 102 may accordingly use different display formats for patient tiles 114 in the dashboard 112 in order to visually signal the acuity levels 116 of individual patients 106, and/or relative differences between acuity levels 116 of different patients 106. For instance, as discussed further below with respect to FIG. 2, patient tiles 114 for patients 106 associated with higher acuity levels 116 may have larger dimensions and/or may be more detailed than patient tiles 114 for other patients 106 who are associated with lower acuity levels 116. Accordingly, a viewer of the dashboard 112 may use visual differences, such as differing dimensions and/or detail levels, between different patient tiles 114 presented concurrently in the dashboard 112 to, at a glance, quickly get a sense of relative differences between acuity levels 116 of corresponding patients 106.

As an example, if the acuity level 116A of the first patient 106A is higher than the acuity level 116B of the second patient 106B, the display format determiner 118 may determine that the first patient tile 114A associated with the first patient 106A should have larger dimensions and/or be more detailed in the dashboard 112 than the second patient tile 114B associated with the second patient 106B. Accordingly, a viewer of the dashboard 112 may quickly determine at a glance, based on the larger dimensions and/or higher detail level of the first patient tile 114A relative to the dimensions and/or detail level of the second patient tile 114B, that the first patient 106A associated with the first patient tile 114A has a higher acuity level 116 than the second patient 106B associated with the second patient tile 114B.

A viewer of the dashboard 112 may also use a display format of a particular single patient tile 114 to get a sense of the corresponding patient's acuity level 116. For example, the third patient tile 114C for the third patient 106C may have relatively small dimensions and/or be relatively simple with a low level of detail in the dashboard 112, thereby signaling to a viewer that the third patient 106C has a relatively low acuity level 116.

In some examples, the acuity levels 116 of patients 106 may be displayed directly as numerical values or other data presented via the patient tiles 114. However, a viewer may also discern the acuity levels 116 of the patients 106 based on the display formats of the corresponding patient tiles 114. As an example, if a viewer is relatively far away from a screen in the healthcare environment 104 that is displaying the dashboard 112, the viewer may not have good enough vision to clearly see numerical values of patients' acuity levels 116 that are displayed within the patient tiles 114. However, the viewer may nevertheless be able to see or discern, from that distance, the dimension sizes, detail levels, and/or other display format attributes of the patient tiles 114 that also signify the respective patients' acuity levels 116. For instance, even if a viewer is too far away to read detailed information displayed in the patient tiles 114, the viewer may still be able to see that one patient tile 114 is larger and/or more detailed than another patient tile 114, and thus be able to infer that the patient 106 associated with the larger and/or more detailed patient tile 114 has a higher acuity level 116 than the patient 106 associated with the smaller and/or less detailed patient tile 114.

In some examples, the display format determiner 118 may determine the size and/or dimensions of the patient tiles 114 based on acuity levels 116 of respective patients 106, without regard for whether additional unused display space is available in the dashboard 112. For example, if all of the patients 106 being treated in the healthcare environment 104 have relatively low acuity levels 116, the display format determiner 118 may cause all of the patient tiles 114 for those patients 106 to be displayed with relatively small dimensions within the dashboard 112. Accordingly, rather than increasing the size and/or dimensions of the patient tiles 114 to fill all available display space within the dashboard 112, all of the patient tiles 114 may be presented with relatively small sizes and/or dimensions. A viewer of the dashboard 112 may be able to quickly determine at a glance, based on the relatively small size and/or dimensions of all of the patient tiles 114, that none of the patients 106 have severe conditions or illnesses.

In this example, if the acuity level 116 of one of the patients 106 later increases, the display format determiner 118 may dynamically cause the patient tile 114 corresponding to that patient 106 to be displayed with larger dimensions within the dashboard 112. A viewer of the dashboard 112 may then be able to quickly determine at a glance, based on the larger dimensions of that patient tile 114 relative to the smaller dimensions of the other patient tiles 114 within the dashboard 112, that the patient 106 corresponding to the larger patient tile 114 has a more severe condition or illness than the other patients 106.

The central monitoring system 102 may have an acuity determiner 120 that determines and/or tracks the acuity levels 116 of respective patients 106, such that the display format determiner 118 may use those acuity levels 116 to determine display formats for respective patient tiles 114 in the dashboard 112. In some examples, the acuity determiner 120 may determine acuity levels 116 of respective patients 106 based on user input. For instance, a doctor, nurse, or other staff member at the healthcare environment 104 may determine a patient's acuity level 116 after examining and/or treating the patient 106, and may provide user input to the central monitoring system 102 that indicates that acuity level 116. The acuity determiner 120 may store the user-provided acuity level 116 and/or provide the user-provided acuity level 116 to the display format determiner 118.

However, in other examples, the acuity determiner 120 may automatically determine and/or adjust acuity levels 116 of one or more of the patients 106 without user input, or in addition to user input. For instance, the acuity determiner 120 may be configured to automatically determine a patient's acuity level 116 based on patient monitoring data 110 received from monitoring devices 108, patient medical records, and/or other information.

As an example, the acuity determiner 120 may be configured with mapping rules that associate certain types of patient monitoring data 110, certain types of monitoring devices 108, certain numbers of monitoring devices 108, certain types of values or metrics in patient medical records, and/or other data points with corresponding acuity levels 116. As another example, the acuity determiner 120 may be a machine learning model, such as a neural network, or other type of model or system that is trained or otherwise configured to predict or determine a patient's acuity level 116 based on types of patient monitoring data 110 received for the patient 106, the types of monitoring devices 108 that provide patient monitoring data 110 for the patient 106, one or more types of current and/or historical metrics or data points indicated by the patient's medical record, and/or other factors. Accordingly, the acuity determiner 120 may use one or more types of data associated with a patient 106 to automatically determine, via mapping rules, a machine learning model, and/or other automated systems, the acuity level 116 of the patient 106.

For instance, some types of patient monitoring data 110 and/or monitoring devices 108 may be commonly used in association with almost every patient 106. However, other types of patient monitoring data 110 and/or monitoring devices 108 may be used more often for patients 106 with severe conditions than for other patients 106. As a non-limiting example, an ECG monitor may be likely to be used to monitor a patient 106 who has a severe heart condition, but an ECG monitor may not be commonly used to monitor a patient 106 who is recovering from a broken leg but is otherwise healthy.

As another non-limiting example, continuous patient monitoring data 110, such as continuous data from an ECG monitor, SPO2 monitor, or CO2 monitor, may indicate that a corresponding patient 106 has a severe condition with symptoms that it may be useful to monitor continuously and therefore has a relatively high acuity level 116. However, patient monitoring data 110 that is measured or provided on a non-continuous basis, such as intermittently or infrequently, may indicate that a corresponding patient 106 has a lower acuity level 116, unless continuous patient monitoring data 110 is also received for that patient 106.

The acuity determiner 120 may be configured with rules, machine learning training data, or other data that rank different types of patient monitoring data 110 and/or different types of monitoring devices 108 as being more or less likely to be associated with high acuity levels 116. Accordingly, if patient monitoring data 110 associated with a particular patient 106 includes types of patient monitoring data 110 that configuration data indicates are generally associated with higher acuity levels 116, and/or that are provided by types of monitoring devices 108 that are generally associated with higher acuity levels 116, the acuity determiner 120 may determine that the acuity level 116 of the patient 106 is relatively high. In some examples, the receipt of patient monitoring data 110 generally associated with a high acuity level 116 may cause the display format determiner 118 to determine that a patient has a high acuity level 116, regardless of whether other types of patient monitoring data 110 generally associated with lower acuity levels 116 are also being received in association with that patient 106.

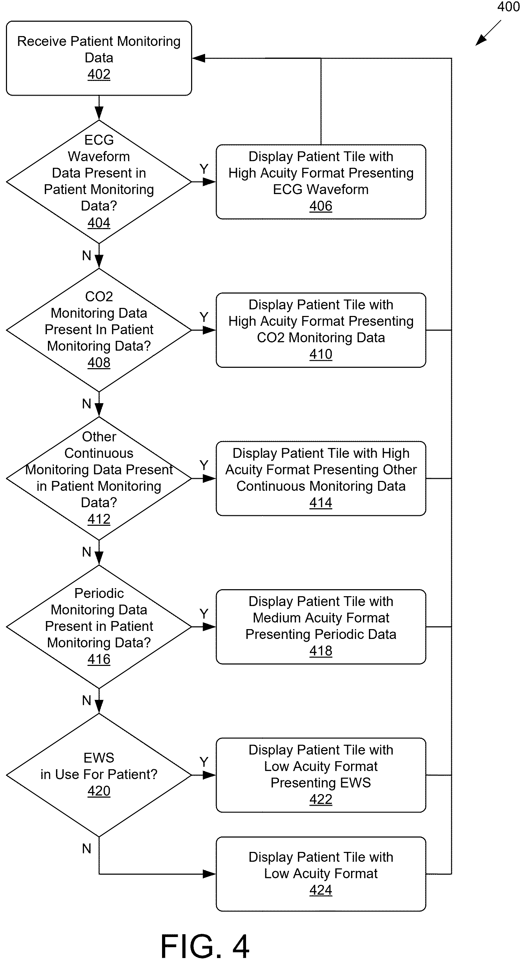

As a non-limiting example, if use of an ECG monitor with a patient 106 is likely to be indicative of the patient 106 having a relatively severe condition, and the acuity determiner 120 determines that patient monitoring data 110 for a particular patient 106 is being provided by an ECG monitor and/or is a type of data associated with an ECG monitor, the acuity determiner 120 may automatically determine that the patient 106 has a relatively high acuity level 116 regardless of whether other types of monitoring devices 108 are also monitoring the patient 106. FIG. 4, described further below, shows another example of using received patient monitoring data 110 to automatically determine an acuity level 116 of a patient 106, and/or to determine a corresponding display format to use for a respective patient tile 114.

Instead of, or in addition to, using received patient monitoring data 110 to automatically determine a patient's acuity level 116, the acuity determiner 120 may be configured to automatically determine the patient's acuity level 116 based on data in the patient's medical record and/or other data about the patient 106. For example, the acuity determiner 120 may have access to medical records or other information stored in one or more databases or other data repositories, such as a hospital information system (HIS), laboratory information system (LIS), or electronic medical record (EMR) system. The acuity determiner 120 may evaluate such records to automatically determine a patient's acuity level 116 based on past diagnoses, past treatments, family medical history, demographic information, and/or other information indicated by the patient's medical records.

In some examples, the acuity determiner 120 may use medical record data or other information to determine whether the healthcare environment 104 is currently using or determining an EWS for a patient 106. If the healthcare environment 104 is not determining an EWS for the patient 106, such that medical staff may have determined that the patient's condition is not likely to worsen in the future, the acuity determiner 120 may determine that the patient 106 has a relatively low acuity level 116 if other information does not indicate a likelihood of a higher acuity level 116 for the patient 106. If the healthcare environment 104 is determining an EWS for the patient 106, the acuity determiner 120 may use that information as a factor, along with other factors, to determine the patient's acuity level 116. As described herein, although an EWS may indicate whether a patient's condition is likely to worsen in the future, the patient's acuity level 116 may indicate the current severity of the patient's condition.

As discussed above, the display format determiner 118 may determine display formats of patient tiles 114 based at least in part on acuity levels 116 of the corresponding patients 106 that are determined by the acuity determiner 120. Accordingly, the display format determiner 118 may determine to display patient tiles 114 corresponding to patients 106 with higher acuity levels 116 with larger dimensions and/or with higher levels of detail, relative to patient tiles 114 corresponding to patients 106 with lower acuity levels 116.

In some examples, the display format determiner 118 may determine display formats of patient tiles 114 based on other factors, instead of or in addition to acuity levels 116 of corresponding patients 106. For example, the display format determiner 118 may determine display formats of patient tiles 114 based on types of monitoring devices 108 being used to monitor corresponding patients 106, amounts of monitoring devices 108 being used to monitor corresponding patients 106, types of patient monitoring data 110 provided by monitoring devices 108 in association with corresponding patients 106, and/or other factors.

The central monitoring system 102 may have a device detector 122 that is configured to identify individual monitoring devices 108 associated with corresponding patients 106, types of monitoring devices 108 associated with corresponding patients 106, amounts of monitoring devices 108 associated with corresponding patients 106, and/or types of patient monitoring data 110 provided by such monitoring devices 108. The device detector 122 may provide such information to the display format determiner 118, such that the display format determiner 118 may use that information to determine display formats for the patient tiles 114.

Patient monitoring data 110 provided by monitoring devices 108 may include one or more identifiers or other data that uniquely identify the monitoring devices 108, indicate types of the monitoring devices 108, indicate manufacturers of the monitoring devices 108, indicate types of information included in the patient monitoring data 110, and/or otherwise indicate attributes of the monitoring devices 108. The device detector 122 may use such information in the patient monitoring data 110 to identify specific monitoring devices 108 and/or types of monitoring devices 108 that provide the patient monitoring data 110 to the central monitoring system 102.

As an example, patient monitoring data 110 may indicate unique identifiers of the corresponding monitoring devices 108, such as Internet Protocol (IP) addresses assigned to the monitoring devices 108, Media Access Control (MAC) addresses of the monitoring devices 108, serial numbers of the monitoring devices 108, and/or other identifiers, network address information, or other attributes. Such information may allow the device detector 122 to identify the monitoring devices 108 and/or distinguish between different monitoring devices 108. For instance, the device detector 122 may distinguish between different monitoring devices 108 to identify how many monitoring devices 108 are associated with each patient 106.

As another example, patient monitoring data 110 may include information that identifies or indicates types of the corresponding monitoring devices 108, such as device type identifiers or manufacturer identifiers. The device detector 122 may use such information to identify the types of the monitoring devices 108 that provide the patient monitoring data 110. For instance, if a particular manufacturer only produces a certain type of monitoring device 108, the device detector 122 may determine that patient monitoring data 110, that includes an identifier of that particular manufacturer, originated from an instance of that certain type of monitoring device 108.

Similarly, the device detector 122 may determine or infer a device type of a monitoring device 108 based on other attributes of patient monitoring data 110. As a first example, if received patient monitoring data 110 includes ECG waveform data, the device detector 122 may determine that the patient monitoring data 110 originated from an ECG monitor. As another example, if pulse rate monitors are often assigned MAC addresses in a particular address range, and patient monitoring data 110 is received from a MAC address in that particular address range, the device detector 122 may determine that a corresponding monitoring device 108 that provided that patient monitoring data 110 is likely to be a pulse rate monitor.

The device detector 122 may also determine patient context information indicating which patients 106 correspond to which monitoring devices 108. In some examples, patient monitoring data 110 provided by a monitoring device 108 to the central monitoring system 102 may include a patient name, medical record number, and/or other patient identifier of a corresponding patient 106, such that the device detector 122 may associate that monitoring device 108 with the identified patient 106. For instance, when a staff member sets up a monitoring device 108 to monitor a patient 106, the staff member may provide input to the monitoring device 108 that configures the monitoring device 108 with a corresponding patient identifier. Accordingly, the monitoring device 108 may include that patient identifier in patient monitoring data 110 provided to the central monitoring system 102, such that the device detector 122 may use the patient identifier to determine which patient 106 is being monitored by the monitoring device 108. In other examples, the central monitoring system 102 may receive user input or other information that associates monitoring devices 108 with corresponding patients 106. For instance, user input to the central monitoring system 102 may indicate serial numbers, device identifiers, network addresses, and/or other identifiers of one or more monitoring devices 108 that have been set up to monitor a particular patient 106, such that the device detector 122 may associate the identified monitoring devices 108 with the particular patient 106.

Overall, the device detector 122 may determine which types of monitoring devices 108 are associated with each patient 106, how many types of monitoring devices 108 are associated with each patient 106, and/or other information about the sets of monitoring devices 108 associated with each patient 106. The device detector 122 may also dynamically determine when monitoring devices 108 are added or removed in association with corresponding patients 106. As a non-limiting example, the device detector 122 may be configured to continuously evaluate patient monitoring data 110 received by the central monitoring system 102 to dynamically determine, substantially in real-time, that newly-detected monitoring devices 108 have begun monitoring corresponding patients 106, and/or that previously-detected monitoring devices 108 that were previously associated with corresponding patients 106 are no longer providing patient monitoring data 110 and may no longer be monitoring those patients 106.

In some examples, the acuity determiner 120 may use information provided by the device detector 122 to determine acuity levels 116 of corresponding patients 106. For instance, as discussed above, the acuity determiner 120 may be configured to automatically determine acuity levels 116 of patients 106 based on which types of monitoring devices 108 are being used to monitor the patients 106, how many monitoring devices 108 are being used to monitor the patients 106, types of patient monitoring data 110 provided by such monitoring devices 108, and/or other attributes of the monitoring devices 108. The acuity determiner 120 may determine those attributes based on information determined by the device detector 122. As a non-limiting example, if the acuity determiner 120 is configured to determine that a patient's acuity level 116 is likely to be higher when a higher number of monitoring devices 108 is being used to monitor the patient 106, the acuity determiner 120 may determine the patient's acuity level 116 based at least in part on a count of monitoring devices 108 associated with the patient 106 that is determined by the device detector 122. Similarly, if the acuity determiner 120 is configured to determine a patient's acuity level 116 based on types of monitoring devices 108 associated with the patient 106, the acuity determiner 120 may determine the patient's acuity level 116 based at least in part on classifications of the types of the monitoring devices 108 determined by the device detector 122.

The display format determiner 118 may also, or alternately, use information provided by the device detector 122 to determine display formats of the patient tiles 114. As an example, if the device detector 122 detects that a new monitoring device 108 is being used in association with a particular patient 106, the display format determiner 118 may dynamically adjust the display format of a corresponding patient tile 114 within the dashboard 112, such that the adjusted display format allows the patient tile 114 to present information indicated by patient monitoring data 110 from the new monitoring device 108. For instance, the display format determiner 118 may dynamically adjust a layout of data elements presented within the patient tile 114 to make room for one or more new data elements that correspond to the new monitoring device 108, by moving previously-presented data elements, by re-sizing previously-presented data elements, by hiding previously-presented data elements, and/or by otherwise adjusting the layout of the data elements.

In some examples, the display format determiner 118 may also or additionally adjust the dimensions and/or detail level of the patient tile 114, for instance by increasing the dimensions of the patient tile 114 to make room to display new data elements that correspond to the new monitoring device 108. However, in other examples, the display format determiner 118 may determine the dimensions and/or detail level of the patient tile 114 based on the acuity level 116 of the corresponding patient 106. Accordingly, if the patient's acuity level 116 has not changed despite the addition of the new monitoring device 108, the display format determiner 118 may determine to maintain the previous dimensions and/or detail level of the corresponding patient tile 114 in the dashboard 112. The display format determiner 118 may instead determine to hide, re-size, and/or move previously-displayed data elements within the patient tile 114 to allow one or more new data elements corresponding to the new monitoring device 108 to be displayed without an overall change to the dimensions and/or detail level of the patient tile 114.

Similarly, the display format determiner 118 may determine to maintain the previous layout of the patient tile 114 despite the addition of the new monitoring device 108, and determine not to display any of the new data elements corresponding to the new monitoring device 108 within the patient tile 114. For instance, if a new monitoring device 108 is added to monitor a patient 106, the display format determiner 118 may determine that information in patient monitoring data 110 provided by the new monitoring device 108 is less important or relevant to a viewer of the dashboard 112 than information in patient monitoring data 110 provided by one or more other monitoring devices 108 associated with the patient 106. Accordingly, the display format determiner 118 may determine not to adjust the display format of the patient tile 114 to present information from patient monitoring data 110 provided by the new monitoring device 108. However, the display format determiner 118 and/or other elements of the central monitoring system 102 may enable options in the patient tile 114, the dashboard 112, or the user interface that allow a user to optionally choose to view information from patient monitoring data 110 received from the new monitoring device 108, and/or from other monitoring devices 108, that is not presented within the patient tile 114.

In some examples, the central monitoring system 102 may cause a patient tile 114 or other user interface elements to enable and/or present user-selectable options or controls that are associated with corresponding monitoring devices 108 detected and/or identified by the device detector 122. For example, if the device detector 122 determines that a new monitoring device 108 has been added to monitor a patient 106, the display format determiner 118 may adjust the display format of a corresponding patient tile 114 to present icons or user-selectable options that allow a user to configure or control the newly-detected monitoring device 108 via the central monitoring system 102.

As an example, when the new monitoring device 108 is first detected in association with the patient 106, a configuration icon may be dynamically added to the corresponding patient tile 114. Such a configuration icon may be selected by a user of the central monitoring system 102 in order to configure which types of patient monitoring data 110 the new monitoring device 108 provides to the central monitoring system 102, how often the new monitoring device 108 provides patient monitoring data 110 to the central monitoring system 102, and/or other operational attributes of the new monitoring device 108. Similarly, when the new monitoring device 108 is first detected in association with the patient 106, control options may be dynamically added to the corresponding patient tile 114 that allow a user of the central monitoring system 102 to remotely control operations of the new monitoring device 108, for instance to cause the new monitoring device 108 to perform operations in response to user commands, to disable alerts or alarms associated with the new monitoring device 108, and/or to control other operational aspects of the new monitoring device 108.

Overall, the central monitoring system 102 may dynamically determine display formats of patient tiles 114 presented within the dashboard 112, based on factors such as acuity levels 116 of the patients 106, types of monitoring devices 108 and/or patient monitoring data 110 associated with the patients 106, amounts of monitoring devices 108 associated with the patients 106, and/or other factors. The central monitoring system 102 may dynamically adjust the display formats of the patient tiles 114 over time, for instance as changes to the patients' acuity levels 116 and/or other factors occur. For example, if a particular patient 106 has a relatively low acuity level 116 at a first time, the display format determiner 118 may determine that the dashboard 112 is to display a corresponding patient tile 114 with relatively small dimensions and/or a relatively low level of detail. However, if at a second time that particular patient's condition has worsened, and the acuity determiner 120 has determined that the acuity level 116 of the patient 106 has increased, the display format determiner 118 may cause the dashboard 112 to dynamically increase the dimensions and/or detail level of the corresponding patient tile 114 to signify the increased acuity level 116 of the patient 106 to a viewer. Examples of such differing display formats for patient tiles 114 are shown in FIG. 2, discussed further below.

FIG. 2 shows examples of different display formats 200 that may be used to present patient tiles 114 within the dashboard 112. The central monitoring system 102 may dynamically determine which display format to use to display each patient tile 114. Different display formats 200 may be associated with different sizes of the patient tiles 114, different dimensions of the patient tiles 114, different detail levels of the patient tiles 114, different data types and/or data elements to be displayed within the patient tiles 114, and/or other differing attributes of the visual presentation of the patient tiles 114 within the dashboard 112.

For example, as shown in FIG. 2, display formats 200 may include high acuity level display formats 202, medium acuity level display formats 204, and/or low acuity level display formats 206. Display formats 200 may also, or alternately, include other display formats that are not shown in FIG. 2.

The high acuity level display formats 202 may be associated with larger dimensions than the medium acuity level display formats 204 and the low acuity level display formats 206. Accordingly, a viewer may be able to discern at a glance, based on the large dimensions of patient tiles 114 presented using the high acuity level display formats 202 and/or relative differences between those dimensions and dimensions of other patient tiles 114, that the corresponding patients 106 have high acuity levels 116.

The high acuity level display formats 202 may also have higher levels of detail that present more types of data, more detailed data, and/or more frequently updated data than the medium acuity level display formats 204 and low acuity level display formats 206. For example, the high acuity level display formats 202 may present continuous monitoring data that is likely to be important to monitor via the dashboard 112 for patients 106 associated with higher acuity levels 116, such as continuous data from an ECG monitor, SPO2 monitor, and/or CO2 monitor. The high acuity level display formats 202 may present such continuous monitoring data from a monitoring device 108 alone, in combination with other continuous monitoring data from one or more other monitoring devices 108, and/or in combination with other data such as vital sign metrics, EWS values, or other values that are continuously or intermittently updated. A viewer may be able to discern at a glance, based on the high detail levels of patient tiles 114 presented using the high acuity level display formats 202, that the corresponding patients 106 have high acuity levels 116.

The medium acuity level display formats 204 may be associated with dimensions that are smaller than the high acuity level display formats 202, but that are larger than the low acuity level display formats 206. Accordingly, a viewer may be able to discern at a glance, based on the medium-sized dimensions of patient tiles 114 presented using the medium acuity level display formats 204 and/or relative differences between those dimensions and dimensions of other patient tiles 114, that the corresponding patients 106 have medium acuity levels 116.

The medium acuity level display formats 204 may also have moderate levels of detail that present more types of data, more detailed data, and/or more frequently updated data than the low acuity level display formats 206, but are less detailed and/or are updated less frequently than the high acuity level display formats 202. As an example, the medium acuity level display formats 204 may not present continuous monitoring data, but may present vital sign metrics, EWS values, or other values that are continuously or intermittently updated. As another example, the medium acuity level display formats 204 may present graphs that display values of one or more types of metrics measured at intermittent intervals over a recent period of time. Accordingly, a viewer may view the graphs to determine trends indicating how those intermittently-determined metrics have been changing over time. A viewer may be able to discern at a glance, based on the moderate detail levels of patient tiles 114 presented using the medium acuity level display formats 204, that the corresponding patients 106 have medium acuity levels 116.

The low acuity level display formats 206 may be associated with dimensions that are smaller than the high acuity level display formats 202 and the medium acuity level display formats 204. Accordingly, a viewer may be able to discern at a glance, based on the small dimensions of patient tiles 114 presented using the low acuity level display formats 206 and/or relative differences between those dimensions and dimensions of other patient tiles 114, that the corresponding patients 106 have low acuity levels 116.

The low acuity level display formats 206 may also have low levels of detail that present fewer types of data, less detailed data, and/or less frequently updated data than the high acuity level display formats 202 and the medium acuity level display formats 204. As an example, the low acuity level display formats 206 may present EWS values and/or charts indicating how such EWS values have been changing over time, but may omit other types of metrics and data types. A viewer may be able to discern at a glance, based on the low detail levels of patient tiles 114 presented using the low acuity level display formats 206, that the corresponding patients 106 have low acuity levels 116.

As described above, a patient's EWS value may be different than the patient's acuity level 116 that indicates a current severity of the patient's condition, and may instead indicate whether the patient's condition is likely to worsen in the future. If a patient 106 has a relatively high EWS due to a high risk of the patient's condition worsening in the future, but the patient's condition is currently not severe, the patient may currently have a low acuity level 116. Accordingly, due to the current low acuity level 116 of the patient 106, the corresponding patient tile 114 may be displayed using a low acuity level display format 206 even though the patient tile 114 may indicate a relatively high EWS for the patient 106.

The central monitoring system 102 may dynamically determine which display formats to use for individual patient tiles 114 based on the acuity levels 116 of the corresponding patients 106, and in some examples also based on additional factors such as the types of monitoring devices 108 that are providing patient monitoring data 110 associated with the patients 106. The central monitoring system 102 may also dynamically change a display format used to present an individual patient tile 114 over time, for instance as the acuity level of the corresponding patient 106 changes and/or other factors associated with the patient change.

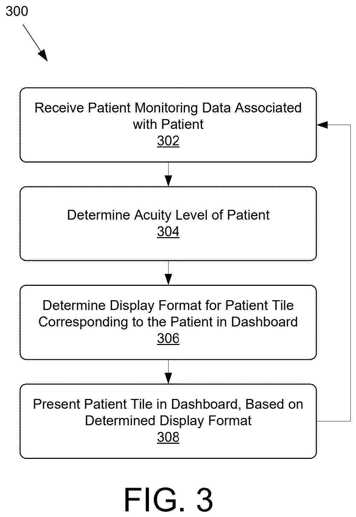

As an example, at a first time a patient 106 may have a low acuity level 116, such that a low acuity level display format 206 is used to display a corresponding patient tile 114 in the dashboard 112. However, at a later second time the patient's acuity level 116 may increase, such that the central monitoring system 102 switches to using a medium acuity level display format 204 or a high acuity level display format 202 to present the patient tile 114 for the patient 106 in the dashboard 112. As another example, at a first time a patient 106 with a high acuity level 116 may be monitored by a set of monitoring devices 108 that provides one type of continuous monitoring data, such that a high acuity level display format 202 that presents one graph of continuous monitoring data may be used to display the corresponding patient tile 114. At a later second time an additional monitoring device 108 that provides an additional type of continuous monitoring data may be added to monitor the patient 106. Accordingly, the central monitoring system 102 may switch to using a different high acuity level display format 202, that presents two graphs of continuous monitoring data, to display the corresponding patient tile 114. Additional examples of dynamically determining and/or adjusting display formats for patient tiles 114 within the dashboard 112 are discussed further below with respect to FIGS. 3-5.

FIG. 3 shows a flowchart illustrating an example method 300 for dynamically determining a display format for a patient tile 114 in the dashboard 112 of the central monitoring system 102. The method 300 shown in FIG. 3 may be performed by a computing system that executes the display format determiner 118 and/or other elements of the central monitoring system 102. An example system architecture for such a computing system is described below with respect to FIG. 6.

At block 302, the computing system may receive patient monitoring data 110 associated with a patient 106. The computing system may receive the patient monitoring data 110 from one or more monitoring devices 108 that have been configured to monitor the patient 106 within the healthcare environment 104. The computing system may use the device detector 122 and/or other elements of the central monitoring system 102 to determine identities and/or types of the monitoring devices 108 that provided the patient monitoring data 110, determine types of information or data elements provided within the patient monitoring data 110, and/or identify which patient 106 is associated with the patient monitoring data 110.

At block 304, the computing system may determine an acuity level 116 of the patient 106. In some examples, the computing system may look up a previously-determined acuity level 116 of the patient 106, for example based on a manual determination of the acuity level by one or more staff members within the healthcare environment 104. However, in other examples the computing system may use the acuity determiner 120 and/or other elements of the central monitoring system 102 to automatically determine the acuity level 116 of the patient 106, based on factors such as the types of monitoring devices 108 that provided the patient monitoring data 110, types of information or data elements provided within the patient monitoring data 110, information indicated in patient medical records or other patient data, and/or other factors.

At block 306, the computing system may determine the display format to use to display a patient tile 114 associated with the patient within the dashboard 112 of the central monitoring system 102. In some examples, the computing system may determine the display format based at least in part on the acuity level 116 of the patient 106 determined at block 304. For example, if the patient's acuity level 116 is high, the computing system may determine to use a high acuity level display format 202 to display the corresponding patient tile 114 within the dashboard 112.

In some examples, the computing system may also, or alternately, use other factors to determine the display format for the patient tile 114 at block 306. For example, the computing system may determine the display format based on types of the monitoring devices 108 that provided the patient monitoring data 110, based on how many monitoring devices 108 provided the patient monitoring data 110, based on types of the patient monitoring data 110, and/or based on other factors instead of or in addition to the patient's acuity level 116.

In some examples, the computing system may determine the display format for the patient tile 114 at block 306 in part by determining which data types to display within the patient tile 114. For example, if the patient's acuity level 116 is low, the computing system may determine to display the patient tile 114 using a low acuity level display format 206 that has relatively small dimensions and has a low level of detail that presents only one or two types of data elements. Accordingly, if the received patient monitoring data 110 indicates multiple data types, the computing system may determine which one or two of those data types to display within the patient tile 114, and which data types to hide from being displayed within the patient tile 114.

At block 308, the computing system may use the display format determined at block 306 to display the patient tile 114 within the dashboard 112. The computing system may return to block 302 to receive additional patient monitoring data 110 associated with the patient 106, and may repeat the process shown in FIG. 3. Accordingly, if the different types of patient monitoring data 110 are received, the patient's acuity level 116 has changed, and/or other changes have occurred during the next iteration of the process shown in FIG. 3, the computing system may potentially select a different display format for the corresponding patient tile 114 at block 306 and dynamically switch the display format of the patient tile 114 within the dashboard 112 at block 308.

The computing system may perform the process shown in FIG. 3 for different patients 106 and patient tiles 114 separately and/or in parallel. Accordingly, the computing system may use the process of FIG. 3 to dynamically determine and adjust display formats for different patient tiles 114 independently.

FIG. 4 shows a flowchart illustrating an example method 400 for dynamically determining a display format for a patient tile 114 in the dashboard 112, based at least in part on types of patient monitoring data 110 associated with a corresponding patient 106. The method 400 shown in FIG. 4 may be performed by a computing system that executes the display format determiner 118 and/or other elements of the central monitoring system 102. An example system architecture for such a computing system is described below with respect to FIG. 6.

At block 402, the computing system may receive patient monitoring data 110 associated with a patient 106. The computing system may receive the patient monitoring data 110 from one or more monitoring devices 108 that have been configured to monitor the patient 106 within the healthcare environment 104. The computing system may use the device detector 122 and/or other elements of the central monitoring system 102 to determine identities and/or types of the monitoring devices 108 that provided the patient monitoring data 110, determine types of information or data elements provided within the patient monitoring data 110, and/or identify which patient 106 is associated with the patient monitoring data 110.

The computing system may be configured with rankings, mapping rules, machine learning systems, and/or other data that associate types of patient monitoring data 110, types of monitoring devices 108, and/or other information with corresponding acuity levels 116 and/or display formats for patient tiles 114. Accordingly, the computing system may evaluate the patient monitoring data 110 received at block 402 to determine whether the received patient monitoring data 110 includes data elements that are indicative of acuity levels 116 associated with corresponding display formats. For example, as shown in FIG. 4, the computing system may be configured with rankings indicating that the presence of continuous ECG waveform data is indicative of a highest acuity level 116, the presence of continuous CO2 data (without higher-ranked data) is indicative of a second-highest acuity level 116, the presence of other continuous monitoring data (without higher-ranked data) is indicative of a third-highest acuity level 116, the presence of periodic monitoring data (without higher-ranked data) is indicative of a fourth-highest acuity level 116, the use of an EWS for the patient (without higher-ranked data) is indicative of a fifth-highest acuity level 116, and that an EWS not being used for the patient (without higher-ranked data) is indicative of a lowest acuity level 116.

Accordingly, at block 404, the computing system may determine whether the patient monitoring data 110 received at block 402 includes ECG waveform data, such as continuous ECG waveform data. If the patient monitoring data 110 includes ECG waveform data (Block 404 - Yes), and the presence of that type of data may indicate that the patient 106 is likely to have the highest acuity level 116, the central monitoring system 102 may determine at block 406 to display the corresponding patient tile 114 using a high acuity level display format 202 that presents the ECG waveform data. If other types of lower-ranked data elements are also present in the patient monitoring data 110, such as other continuous monitoring data, periodic monitoring data, and/or other types of data, the computing system may determine whether to display any of those other data elements within the patient tile 114 alongside the ECG waveform data. The computing system may also return to block 402 to receive additional patient monitoring data 110 and repeat the process of FIG. 4, such that the computing system may select a different display format for the patient tile 114 if the types of patient monitoring data 110 received for the patient 106 change in the future.

If the patient monitoring data 110 does not include ECG waveform data (Block 404 - No), the computing system may determine at block 408 whether the patient monitoring data 110 includes CO2 monitoring data, such as continuous CO2 monitoring data. If the patient monitoring data 110 includes CO2 monitoring data (Block 408 - Yes), and the presence of that type of data (without the presence of higher-ranked data such as ECG waveform data) may indicate that the patient 106 is likely to have the second-highest acuity level 116, the central monitoring system 102 may determine at block 410 to display the corresponding patient tile 114 using a high acuity level display format 202 that presents the CO2 monitoring data. If other types of lower-ranked data elements are also present in the patient monitoring data 110, such as other continuous monitoring data, periodic monitoring data, and/or other types of data, the computing system may determine whether to display any of those other data elements within the patient tile 114 alongside the CO2 monitoring data. The computing system may also return to block 402 to receive additional patient monitoring data 110 and repeat the process of FIG. 4, such that the computing system may select a different display format for the patient tile 114 if the types of patient monitoring data 110 received for the patient 106 change in the future.

If the patient monitoring data 110 does not include CO2 monitoring data (Block 408 - No), the computing system may determine at block 412 whether the patient monitoring data 110 includes one or more other types of continuous monitoring data that are different from ECG waveform data and CO2 monitoring data, such as continuous SPO2 monitoring data, continuous pulse rate data, and/or other types of continuous monitoring data. If the patient monitoring data 110 includes any such types of other continuous monitoring data (Block 412 - Yes), and the presence of that type of data (without the presence of higher-ranked data such as ECG waveform data or CO2 monitoring data) may indicate that the patient 106 is likely to have the third-highest acuity level 116, the central monitoring system 102 may determine at block 414 to display the corresponding patient tile 114 using a high acuity level display format 202 that presents the other continuous monitoring data. If other types of lower-ranked data elements are also present in the patient monitoring data 110, such as periodic monitoring data and/or other types of data, the computing system may determine whether to display any of those other data elements within the patient tile 114 alongside the other continuous monitoring data. The computing system may also return to block 402 to receive additional patient monitoring data 110 and repeat the process of FIG. 4, such that the computing system may select a different display format for the patient tile 114 if the types of patient monitoring data 110 received for the patient 106 change in the future.

If the patient monitoring data 110 does not include other continuous monitoring data (Block 412 - No), the computing system may determine at block 416 whether the patient monitoring data 110 includes one or more types of periodic monitoring data, such as types of data that are measured on an intermittent basis at regular intervals or on an irregular schedule. If the patient monitoring data 110 includes any such types of periodic monitoring data (Block 416 - Yes), and the presence of that type of data (without the presence of higher-ranked continuous data) may indicate that the patient 106 is likely to have the fourth-highest acuity level 116, the central monitoring system 102 may determine at block 418 to display the corresponding patient tile 114 using a medium acuity level display format 204 that presents one or more data elements indicated by the periodic monitoring data. If other types of lower-ranked data elements are also present in the patient monitoring data 110, such as EWS data or other data elements, the computing system may determine whether to display any of those other data elements within the patient tile 114 alongside the periodic monitoring data elements. The computing system may also return to block 402 to receive additional patient monitoring data 110 and repeat the process of FIG. 4, such that the computing system may select a different display format for the patient tile 114 if the types of patient monitoring data 110 received for the patient 106 change in the future.

If the patient monitoring data 110 does not include periodic monitoring data (Block 416 - No), the computing system may determine at block 420 whether the healthcare environment 104 is using an EWS for the patient 106. For example, the computing system may evaluate the patient monitoring data 110 and/or other information, such as medical records, to determine whether an EWS is in use for the patient 106. If the healthcare environment 104 is using an EWS for the patient 106 (Block 420 - Yes), the computing system may determine at block 422 to display the corresponding patient tile 114 using a low acuity level display format 202 that presents the EWS. In some examples, the computing system may also determine whether to display any other lower-ranked data elements within the patient tile 114 alongside the EWS, such as a patient name or identifier. If the healthcare environment 104 is not using an EWS for the patient 106 (Block 420 - No), the computing system may determine at block 424 to display the corresponding patient tile 114 using a low acuity level display format 202 that does not present an EWS. In some examples, the computing system may also determine whether to display any lower-ranked data elements within the patient tile 114, such as a patient name or identifier. After determining the display format at either block 422 or block 424, the computing system may also return to block 402 to receive additional patient monitoring data 110 and repeat the process of FIG. 4, such that the computing system may select a different display format for the patient tile 114 if the types of patient monitoring data 110 received for the patient 106 change in the future.

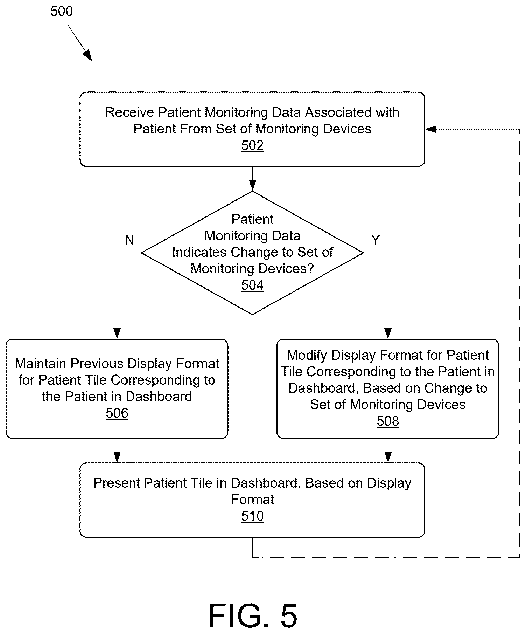

FIG. 5 shows a flowchart illustrating an example method 500 for dynamically determining a display format for a patient tile 114 in the dashboard 112, based at least in part on changes to a set of monitoring devices 108 associated with a corresponding patient 106. The method 500 shown in FIG. 5 may be performed by a computing system that executes the display format determiner 118 and/or other elements of the central monitoring system 102. An example system architecture for such a computing system is described below with respect to FIG. 6.

At block 502, the computing system may receive patient monitoring data 110 associated with a patient 106. The computing system may receive the patient monitoring data 110 from a set of one or more monitoring devices 108 that have been configured to monitor the patient 106 within the healthcare environment 104. The computing system may use the device detector 122 and/or other elements of the central monitoring system 102 to determine identities and/or types of the monitoring devices 108 that provided the patient monitoring data 110, determine types of information or data elements provided within the patient monitoring data 110, and/or identify which patient 106 is associated with the patient monitoring data 110.

At block 504, the computing system may determine whether the patient monitoring data 110 received at block 502 indicates that there has been a change to the set of monitoring devices 108 associated with the patient 106, relative to a previously-determined state of the set of monitoring devices 108 associated with the patient 106. As an example, at block 504 the computing system may determine that a new monitoring device 108 has been added to the set of monitoring devices 108 associated with the patient 106. As another example, at block 504 the computing system may determine that a monitoring device 108 that was previously associated with the patient 106 is no longer part of the set of monitoring devices 108 associated with the patient 106. As another example, at block 504 the computing system may determine that a monitoring device 108 associated with the patient 106 has begun providing a new type of patient monitoring data 110 or is providing patient monitoring data 110 on a different schedule. For instance, if a monitoring device 108 had previously been providing patient monitoring data 110 relatively infrequently on an intermittent basis, but begins providing that patient monitoring data 110 or different patient monitoring data 110 on a continuous basis, the computing system may detect that change at block 504.

If the computing system determines that the received patient monitoring data 110 does not indicate a change to the set of monitoring devices 108 associated with the patient 106 (Block 504 - No), the computing system may determine at block 506 to maintain a previously-used and/or most recent display format for the patient tile 114 corresponding to the patient 106 in the dashboard 112. For instance, if there has not been a change to the set of monitoring devices 108 associated with the patient 106, the dimensions and detail level of the patient tile 114 may remain the same in the dashboard 112, even though data displayed within the patient tile 114 may be updated based on the received patient monitoring data 110.

However, if the computing system instead determines that the received patient monitoring data 110 does indicate a change to the set of monitoring devices 108 associated with the patient 106 (Block 504 - Yes), the computing system may determine at block 506 to modify the display format for the patient tile 114 based on the change to the set of monitoring devices 108. As an example, if the set of monitoring devices 108 has been changed because an additional monitoring device 108 has been added to monitor the patient 106, the computing system may modify the display format of the patient tile 114 so that one or more data elements provided by the additional monitoring device 108 may be displayed in the patient tile 114, so that UI controls associated with the additional monitoring device 108 may be presented within the patient tile 114, and/or so that other information associated with the additional monitoring device 108 may be presented within the patient tile 114.

As other examples, if the set of monitoring devices 108 has been changed because a previous monitoring device 108 is no longer associated with the patient 106, or a monitoring device 108 is providing a new type of patient monitoring data 110 or is providing patient monitoring data 110 on a different schedule, the computing system may modify the display format of the patient tile 114 to account for the change. For instance, if a previous monitoring device 108 is no longer associated with the patient 106, the computing system may modify the display format so that the patient tile 114 no longer displays patient monitoring data 110 from that previous monitoring device 108, and/or displays one or more other data elements from one or more other monitoring devices 108 instead. Similarly, if a monitoring device 108 has changed from providing intermittent monitoring data to providing continuous monitoring data, the computing system may adjust the display format so that the patient tile 114 presents the continuous monitoring data instead of the intermittent monitoring data.

In some examples, a change to the set of monitoring devices 108 may indicate a change to the acuity level 116 of the patient 106. For instance, if the change indicates that a new type of monitoring device 108 has been added, or that a monitoring device 108 has changed the type and/or frequency of patient monitoring data 110 it is providing, that change may be indicative of an increase in the patient's acuity level 116. The computing device may accordingly modify the display format at block 508 to change the dimensions and/or detail level of the patient tile 114 based on the change in the patient's acuity level 116, as discussed above. However, in other examples, the change to the set of monitoring devices 108 may lead to a corresponding change in a layout and/or types of data displayed within the patient tile 114, without a change to the dimensions and/or detail level of the patient tile 114. Additionally, while the process shown in FIG. 5 may change the display format of the patient tile 114 based on a corresponding change to the set of monitoring devices 108 associated with the patient 106, the computing device may also change the display format of the patient tile 114 based on other factors. For instance, if the patient's acuity level 116 has changed due to other reasons not related to a change to the set of monitoring devices 108 associated with the patient 106, the computing system may adjust the display format for the patient tile 114 based on the change in the acuity level 116.

At block 510, the computing system may use the display format determined at either block 506 or block 508 to present the patient tile 114 associated with the patient 106 within the dashboard 112. The computing system may also return to block 502 to receive additional patient monitoring data 110 and repeat the process of FIG. 5, such that the computing system may modify or maintain the display format for the patient tile 114 depending on whether the set of monitoring devices 108 associated with the patient 106 changes in the future.

FIG. 6 shows an example system architecture 600 for a computing system 602 that may execute one or more elements described herein, such as one or more elements of the central monitoring system 102. The computing system 602 may include one or more computers, servers, or other types of computing devices. In some examples, monitoring devices 108 may include, or be associated with, computing systems that have the system architecture 600 shown in FIG. 6 or a similar system architecture. In some examples, other computing devices and systems described herein, such as user devices that may be used to access the central monitoring system 102 and/or view the dashboard 112, may also have the system architecture 600 shown in FIG. 6 or a similar system architecture.

In some examples, different elements described herein may be distributed among, and/or be executed by, multiple computing systems or devices similar to the computing system 602 shown in FIG. 6. As an example, the display format determiner 118 may be executed by a different computing system than a computing system that executes the acuity determiner 120.

In some examples, the computing system 602 may include, or be part of, a cloud computing environment or other distributed system that hosts and/or executes one or more elements described herein. For instance, in some examples, the acuity determiner 120 may be executed by a cloud computing environment or other computing environment that is different from a computing environment that separately executes the display format determiner 118 and/or that generates and displays the dashboard 112.

The computing system 602 may include memory 604. In various examples, the memory 604 may include system memory, which may be volatile (such as RAM), non-volatile (such as ROM, flash memory, etc.) or some combination of the two. The memory 604 may further include non-transitory computer-readable media, such as volatile and nonvolatile, removable and non-removable media implemented in any method or technology for storage of information, such as computer readable instructions, data structures, program modules, or other data. System memory, removable storage, and non-removable storage are all examples of non-transitory computer-readable media. Examples of non-transitory computer-readable media include, but are not limited to, RAM, ROM, EEPROM, flash memory or other memory technology, CD-ROM, digital versatile discs (DVD) or other optical storage, magnetic cassettes, magnetic tape, magnetic disk storage or other magnetic storage devices, or any other non-transitory medium which may be used to store desired information and which may be accessed by the computing system 602. Any such non-transitory computer-readable media may be part of the computing system 602.

The memory 604 may store one or more types of data, computer-executable instructions associated with software elements, firmware elements, or other executable elements, and/or other information associated with elements described herein. As an example, the memory 604 may store data and/or computer-executable instructions associated with the central monitoring system 102, such as the dashboard 112, the display format determiner 118, the acuity determiner 120, and/or the device detector 122.

The memory 604 may also store other modules and data 606 that may be utilized by the computing system 602 to perform or enable performing any action taken by the computing system 602. For example, the other modules and data 606 may include a platform, operating system, and/or applications, as well as data utilized by the platform, operating system, and/or applications.

The computing system 602 may also have processor(s) 608, communication interfaces 610, a display 612, output devices 614, input devices 616, and/or a drive unit 618 including a machine readable medium 620.

In various examples, the processor(s) 608 may be a central processing unit (CPU), a graphics processing unit (GPU), both a CPU and a GPU, or any other type of processing unit. Each of the one or more processor(s) 608 may have numerous arithmetic logic units (ALUs) that perform arithmetic and logical operations, as well as one or more control units (CUs) that extract instructions and stored content from processor cache memory, and then execute these instructions by calling on the ALUs, as necessary, during program execution. The processor(s) 608 may also be responsible for executing computer applications stored in the memory 604, which may be associated with types of volatile (RAM) and/or nonvolatile (ROM) memory.

The communication interfaces 610 may include transceivers, modems, network interfaces, antennas, and/or other components that may transmit and/or receive data over networks or other connections. In some examples, the communication interfaces 610 may be used to exchange data between elements described herein. As an example, the communication interfaces 610 may be used by the central monitoring system 102 to receive patient monitoring data 110 from one or more monitoring devices 108. As another example, the communication interfaces 610 may allow a user device to access and interact with the dashboard 112 of the central monitoring system 102, for instance via the Internet or another network connection.

The display 612 may be a liquid crystal display, or any other type of display commonly used in computing devices. For example, a display 612 may be a touch-sensitive display screen, and can then also act as an input device or keypad, such as for providing a soft-key keyboard, navigation buttons, or any other type of input. In some examples, the dashboard 112 may be presented via the display 612.

The output devices 614 may include any sort of output devices known in the art, such as the display 612, speakers, a vibrating mechanism, and/or a tactile feedback mechanism. Output devices 614 may also include ports for one or more peripheral devices, such as headphones, peripheral speakers, and/or a peripheral display.

The input devices 616 may include any sort of input devices known in the art. For example, input devices 616 may include a microphone, a keyboard/keypad, and/or a touch-sensitive display, such as a touch-sensitive display screen. A keyboard/keypad may be a push button numeric dialing pad, a multi-key keyboard, or one or more other types of keys or buttons, and may also include a joystick-like controller, designated navigation buttons, or any other type of input mechanism.

The machine readable medium 620 may store one or more sets of instructions, such as software or firmware, that embody any one or more of the methodologies or functions described herein. The instructions may also reside, completely or at least partially, within the memory 604, processor(s) 608, and/or communication interface(s) 610 during execution thereof by the computing system 602. The memory 604 and the processor(s) 608 also may constitute machine readable media 620.

Although the subject matter has been described in language specific to structural features and/or methodological acts, it is to be understood that the subject matter is not necessarily limited to the specific features or acts described above. Rather, the specific features and acts described above are disclosed as example embodiments.

Embodiments of the invention can be described with reference to the following numbered clauses, with preferred features laid out in the dependent clauses:
1 A dashboard, presented via a user interface of a central monitoring system associated with a healthcare environment, comprising:
   a first patient tile, associated with a first patient, wherein:
      the first patient tile is configured to present first data elements indicated by first patient monitoring data received by the central monitoring system from at least one first monitoring device associated with the first patient within the healthcare environment, and
      the first patient tile is displayed within the dashboard using a first display format dynamically determined by a computing system associated with the central monitoring system based at least in part on a first acuity level of the first patient; and
   a second patient tile, associated with a second patient, wherein:
      the second patient tile is configured to present second data elements indicated by second patient monitoring data received by the central monitoring system from at least one second monitoring device associated with the second patient within the healthcare environment, and
      the second patient tile is displayed within the dashboard using a second display format dynamically determined by the computing system based at least in part on a second acuity level of the second patient.
2 The dashboard of clause 1, wherein the first acuity level is automatically determined by the central monitoring system based on indications, by the first patient monitoring data, of at least one of:
   a device type of the at least one first monitoring device,
   a data type of the first patient monitoring data, or
   a monitoring frequency of the first patient monitoring data.
3 The dashboard of either clause 1 or clause 2, wherein:
   the first acuity level of the first patient is higher than the second acuity level of the second patient, and
   the first display format of the first patient tile, determined based on the first acuity level, indicates at least one of larger dimensions or a higher detail level for the first patient tile, relative to smaller dimensions or a lower detail level for the second patient tile indicated by the second display format determined based on the second acuity level.
4 The dashboard of any preceding clause, wherein the first data elements presented in the first patient tile comprise a set of data elements received from a plurality of monitoring devices associated with the first patient.

## Claims

1. A central monitoring system associated with a healthcare environment, comprising:
a dashboard, presented in a user interface displayed via a computing system comprising a processor, configured to display a plurality of patient tiles, wherein:
the plurality of patient tiles is associated with a plurality of patients present within the healthcare environment, and
an individual patient tile of the plurality of patient tiles:
corresponds to an individual patient of the plurality of patients, and
displays data elements indicated by patient monitoring data provided by at least one monitoring device associated with the individual patient within the healthcare environment; and
a display format determiner, executed by the computing system, configured to dynamically determine display formats used to display the plurality of patient tiles in the dashboard,
wherein the display format determiner determines a display format for the individual patient tile based at least in part on an acuity level of the individual patient.

2. The central monitoring system of claim 1, further comprising an acuity determiner, executed by the computing system, that is configured to automatically determine the acuity level of the individual patient based on the patient monitoring data provided by the at least one monitoring device associated with the individual patient.

3. The central monitoring system of claim 2, wherein the acuity determiner automatically determines the acuity level based on a device type of the at least one monitoring device.

4. The central monitoring system of either claim 2 or claim 3, wherein the acuity determiner automatically determines the acuity level based on a data type of the patient monitoring data provided by the at least one monitoring device.

5. The central monitoring system of any one of claims 2 to 4, wherein the acuity determiner automatically determines the acuity level based on a monitoring frequency of the patient monitoring data provided by the at least one monitoring device.

6. The central monitoring system of any preceding claim, wherein the display format indicates at least one of dimensions or a detail level of the individual patient tile within the dashboard.

7. The central monitoring system of claim 6, wherein:
the display format indicating at least one of larger dimensions or higher detail levels is indicative of the acuity level being higher, and
the display format indicating at least one of smaller dimensions or lower detail levels is indicative of the acuity level being lower.

8. The central monitoring system of either claim 6 or claim 7, wherein the display format further indicates at least one of a selection or a layout of the data elements displayed within the individual patient tile.

9. The central monitoring system of any preceding claim, wherein the display format determiner is further configured to dynamically change the display format of the individual patient tile within the dashboard based on a change to the acuity level of the individual patient.

10. The central monitoring system of any preceding claim, wherein the display format determiner is further configured to dynamically change the display format of the individual patient tile within the dashboard based on a change to a set of monitoring devices associated with the individual patient within the healthcare environment.

11. The central monitoring system of claim 10, wherein the change to the set of monitoring devices comprises at least one of:
an adjusted amount of monitoring devices in the set of monitoring devices,
an adjusted type of data provided by the set of monitoring devices, or
an adjusted monitoring frequency associated with the patient monitoring data.

12. A computer-executed method comprising:
receiving, by a central monitoring system executed by a computing system comprising a processor, patient monitoring data associated with a patient in a healthcare environment from at least one monitoring device;
determining, by the central monitoring system, an acuity level of the patient, wherein the acuity level indicates a current severity of at least one of a medical condition or illness of the patient;
determining, by the central monitoring system, and based at least in part on the acuity level of the patient, a display format for a patient tile associated with the patient that presents at least one data element indicated by the patient monitoring data; and
causing, by the central monitoring system, the patient tile to be displayed in a user interface dashboard of the central monitoring system based on the display format.

13. The computer-executed method of claim 12, further comprising:
receiving, by the central monitoring system, additional patient monitoring data associated with additional patients in the healthcare environment from additional monitoring devices;
determining, by the central monitoring system, respective acuity levels of the additional patients;
determining, by the central monitoring system, and based at least in part on the respective acuity levels of the additional patients, respective display formats for respective patient tiles associated with the additional patients; and
causing, by the central monitoring system, the respective patient tiles to be displayed in the user interface dashboard based on the respective display formats.

14. The computer-executed method of either claim 12 or claim 13, wherein determining the acuity level of the patient comprises determining, based on the patient monitoring data, at least one of:
a device type of the at least one monitoring device,
a data type of the patient monitoring data, or
a monitoring frequency of the patient monitoring data.

15. The computer-executed method of any one of claims 12 to 14, further comprising:
receiving, by the central monitoring system, additional patient monitoring data associated with the patient from the at least one monitoring device;
determining, by the central monitoring system, a change to the acuity level of the patient;
determining, by the central monitoring system, and based at least in part on the change to the acuity level of the patient, a modified display format for the patient tile that presents at least one second data element indicated by the additional patient monitoring data; and
causing, by the central monitoring system, the patient tile to be displayed in the user interface dashboard based on the modified display format, and/determining, by the central monitoring system, a change to a set of monitoring devices associated with the patient, wherein the change comprises at least one of:
an adjusted amount of monitoring devices in the set of monitoring devices,
an adjusted type of data provided by the set of monitoring devices, or
an adjusted monitoring frequency associated with the patient monitoring data;
determining, by the central monitoring system, and based at least in part on the change, a modified display format for the patient tile; and
causing, by the central monitoring system, the patient tile to be displayed in the user interface dashboard based on the modified display format.
